# EUROPEAN PATENT APPLICATION

(11) **EP 4 696 290 A1**
(43) Date of publication of application: **18.02.2026**
(21) Application number: 25189396.2
(22) Date of filing: 14.07.2025
(51) Int. Cl.: A61F 9/007, A61M 1/00

(54) **A HANDPIECE FOR USE IN A SURGICAL PROCEDURE**

(30) Priority: 26.07.2024 NL 2038344
(71) Applicant: YoungEye Medical B.V., 5688 GA Oirschot (NL)
(72) Inventor: Jongelie, Michael Jozef Adriaan, 7071 PC Ulft (NL)
(74) Representative: 't Jong, Bastiaan Jacob

(57) **Abstract**

The invention relates to a handpiece for use in a surgical procedure such as a phacoemulsification, which handpiece comprises a housing with a proximal and a distal outer end, wherein the handpiece comprises at the distal outer end an aspiration opening for aspirating a surgery site, which aspiration opening is connected to an aspiration channel extending through the housing and which aspiration channel is configured to be connected to a suction force source such as a pump or underpressure system in order to generate a suction force in the aspiration opening, wherein the handpiece comprises an operating element, wherein in a first operative mode of the operating element there is suction force in the aspiration opening and in a second operative mode of the operating element there is no suction force in the aspiration opening.

## Description

The present invention relates to a handpiece for use in a surgical procedure such as a phacoemulsification, which handpiece comprises a housing with a proximal and a distal outer end, wherein the handpiece comprises at the distal outer end an aspiration opening for aspirating a surgery site, which aspiration opening is connected to an aspiration channel extending through the housing and which aspiration channel is configured to be connected to a suction force source such as a pump or underpressure system in order to generate a suction force in the aspiration opening.

Such handpieces are frequently used in for instance cataract surgeries. In the case of cataracts the lens of the eye is clouded, which results in decreased sight. A frequently applied treatment is a surgical procedure named phacoemulsification. In phacoemulsification access to the clouded lens of the eye is gained by means of a small incision in the cornea, wherein an opening is also made in the capsular bag of the lens via the incision. Via the opening the phacoemulsification can be carried out by means of ultrasonic vibrations. These vibrations break the lens up into small pieces, which are then removed from the eye using an aspiration handpiece connected to an aspiration device via the aspiration opening of the handpiece.

A drawback of the known bimanual and coaxial handpieces is that there are no intuitive provisions for a quick response for any other tissue which can be drawn in or has already been drawn in via the aspiration opening accidentally.

It is now an object of the invention to reduce or even obviate the above stated drawback.

This object is achieved according to the invention with a handpiece according to the preamble, characterized in that the handpiece comprises an operating element, wherein in a first operative mode of the operating element there is suction force in the aspiration opening and in a second operative mode of the operating element there is no suction force in the aspiration opening.

By providing an operating element on the handpiece itself, whereby the user can control the suction force in the aspiration opening directly, a highly intuitive operation for the user is obtained. Operation need then not be controlled via an external controller of the suction force source, such as for instance a foot pedal, but can take place directly from the handpiece. It is highly preferred that this operation of the suction force is controlled primarily in the handpiece itself, although there can also be signal transmission to the suction force source for the purpose of regulating the suction force.

Between the first and the second operative mode there can optionally be a gradual progression in the suction force in the aspiration opening.

In a preferred embodiment of a handpiece according to the invention the second operative mode is configured for the content of at least a part of the aspiration channel to flow back through the aspiration opening.

Allowing flowing back of at least a part of the aspiration channel through the aspiration opening in the second operative mode of the operating element enables the matter that was suctioned last to flow back into the operating site in simple manner. This makes it possible to react intuitively not only to tissue that appears in danger of being drawn in accidentally via the aspiration opening, but also when it has been drawn in recently.

The aspiration channel is preferably formed by a flexible tube.

The flexible tube can be fed through the handpiece, whereby separate couplings between the handpiece and the tube are not necessary. This simplifies the design of the handpiece, and thereby the production costs.

Another embodiment of a handpiece according to the invention is a handpiece wherein the aspiration channel comprises a shut-off valve connected operatively to the operating element, which shut-off valve opens the aspiration channel in the first operative mode and closes the aspiration channel in the second operative mode.

Connecting a shut-off valve to the operating element enables the aspiration channel to be closed in simple manner in order to thus reduce or even completely stop the suction force in the aspiration opening.

In yet another embodiment of the handpiece according to the invention the shut-off valve presses the flexible tube shut from the outside in the second operative mode.

In this embodiment no part of the handpiece comes into direct contact with the aspiration channel, whereby no contamination can take place of the liquid in the aspiration channel, which is particularly important when a part of the liquid flows back via the aspiration opening.

Also according to the invention is an embodiment of a handpiece wherein in a third operative mode of the operating element there is no suction force in the aspiration opening and an internal volume of the aspiration channel which is connected to the aspiration opening in the third operative mode can be reduced for the purpose of the content of at least a part of the internal volume flowing back through the aspiration opening.

The ability to reduce the internal volume of the aspiration channel in a third operative mode enables flowing back of the content of at least a part of the internal volume through the aspiration opening to take place in forced manner, wherein the user can exert greater control over the volume flowing back and the speed at which it happens.

For this purpose the aspiration channel can for instance comprise a compressible segment, so that the internal volume is reduced, and can optionally also be closed off, by compressing the channel. An element connected operatively to the operating element can for this purpose also be placed into the aspiration channel for the purpose of reducing the internal volume.

In a preferred embodiment of the handpiece according to the invention the operating element comprises a part movable toward the aspiration opening for the purpose of reducing the internal volume of the aspiration channel, which is connected to the aspiration opening in the third operative mode.

When the aspiration channel is closed off in the second operative mode, the volume between the aspiration opening and the shut-off valve can be reduced by the movable part in the third operative mode. A part of the liquid in the aspiration channel can thus flow back again via the aspiration opening.

In a further preferred embodiment of the handpiece according to the invention the movable part is formed by an elongate operating part, which is arranged bendably on the operating element close to the shut-off valve and extends toward the aspiration opening.

With the bendable part the flexible tube can be pressed shut gradually from the shut-off valve to the aspiration opening, whereby the internal volume is gradually reduced and the flowing back via the aspiration opening can be controlled easily.

The elongate operating part preferably has a curved contact surface, whereby the flexible tube can be pressed shut gradually from the shut-off valve toward the aspiration opening by bending of the elongate operating part.

Owing to the curved contact surface, no forces are exerted in the longitudinal direction of the flexible tube but solely perpendicularly of the tube, whereby the force for operating the operating part is used optimally for pressing the tube shut gradually in the direction of the aspiration opening.

In yet another embodiment of a handpiece according to the invention the handpiece also comprises at the distal outer end an irrigation opening for irrigating an operating site, which irrigation opening is connected to an irrigation channel extending through the housing and which irrigation channel is configured to be connected to an irrigation source such as a pump system in order to generate an irrigation flow in the irrigation opening.

By also providing an irrigation option in the handpiece, the handpiece can also be used instead of a known combined handpiece, such as a coaxial handpiece.

These and other features of the invention are further elucidated with reference to the accompanying drawings.
Figure 1 shows a cut-away of a cross-sectional view of a first embodiment of a handpiece according to the invention with the operating element in the first operative mode.
Figure 2 shows the cut-away of figure 1 with the operating element in the third operative mode.
Figure 3 shows a perspective view of a second embodiment of an handpiece according to the invention.
Figure 4 shows a cut-away of a cross-sectional view of the handpiece of figure 3.
Figures 5A - 5C shows a cross-section of a third embodiment of a handpiece according to the invention in three different operative modes.

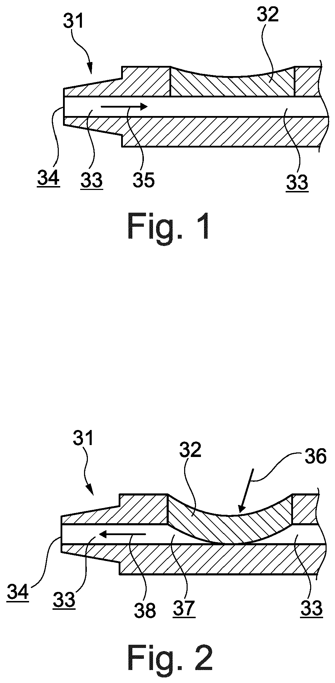

Visible in figure 1 is a cut-away of a cross-sectional view of a first embodiment of a handpiece 31 according to the invention. The operating element 32 takes the form of an elastically deformable segment in the aspiration channel 33. In the shown first operative mode the aspiration channel 33 is opened and a suction force is present at the aspiration opening 34. This creates a flow 35.

As shown in figure 2, the operating element 32 can close the aspiration channel 33 by compression 36. Pressing on the operating element 32 still further after closing reduces the internal volume 37, whereby the flow direction 38 changes and the matter that was just sucked up can flow out through the aspiration opening. Following the second operative mode, the operating element 32 thus enters into the shown third operative mode by further compression.

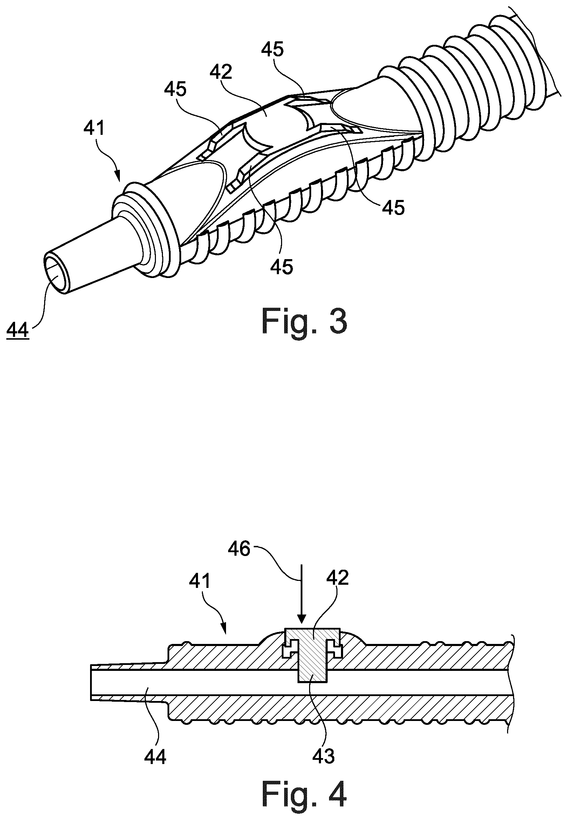

Figure 3 shows a second embodiment of a handpiece 41 according to the invention. The operating element 42 forms a shut-off valve 43, as is clearly visible in the cross-section of figure 4. The shut-off valve 43 is shown in the first operative mode, in which the aspiration channel 44 is opened. The operating element 42 is connected to spring element 45, the outer ends of which are visible. The spring elements 45 provide a force for urging the shut-off valve 43 into the first operative mode.

It is clearly visible in the cross-section in figure 4 that the aspiration channel 44 can be closed by pressing the operating element 42 down in the direction of the arrow 46. The outer end of the shut-off valve 43 or optionally the whole shut-off valve 43 can here be made from a compressible material, so that a third operative mode of the operating element 42 is obtained upon further compression. The deformation of the shut-off valve 43 then reduces here the internal volume of the aspiration channel 44 which is still connected to the aspiration opening in the third operative mode.

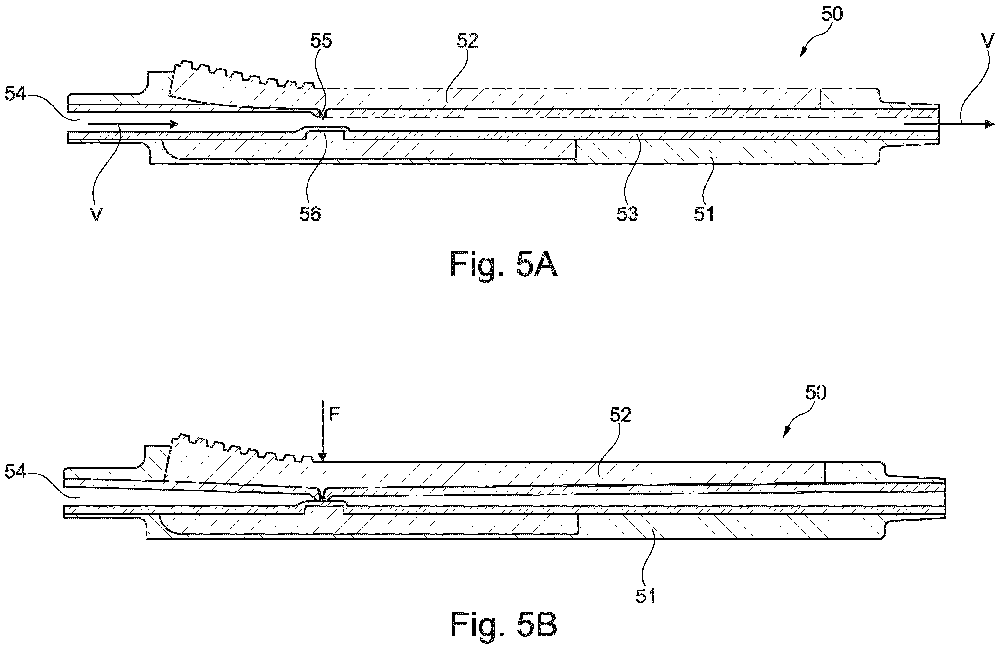

Figure 5A shows a cross-section of a third embodiment of a handpiece 50 according to the invention.

The handpiece 50 has a housing 51 with received therein an operating element 52. A flexible tube 53 extends through the housing 51 and the operating element 52 and forms thereby the aspiration channel with an aspiration opening 54 on one side.

A shut-off valve is formed on the operating element 52 by a sharp edge 55 and a stop surface 56. The flexible tube 53 extends between this sharp edge 55 and stop surface 56.

Figure 5A shows the first operative mode, in which a liquid V can be suctioned out via the aspiration opening 54 and through the aspiration channel formed by the flexible tube 53.

In figure 5B a force F is exerted on operating element 52, whereby the sharp edge 55 presses the flexible tube 53 shut onto the stop surface 56, whereby the suction by the aspiration channel is stopped.

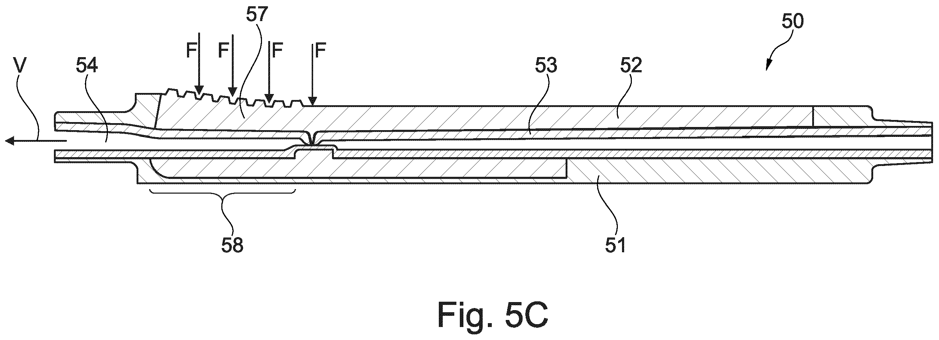

Figure 5C shows the handpiece 50 in the third operative mode. The elongate operating part 57 of the operating element 52 is here bent by an additional force F, whereby the operating element 52 gradually presses the flexible tube 53 shut over a section 58. A part of the suctioned-out liquid V will hereby flow back again via the aspiration opening 54.

## Claims

1. A handpiece for use in a surgical procedure such as a phacoemulsification, which handpiece comprises a housing with a proximal and a distal outer end, wherein the handpiece comprises at the distal outer end an aspiration opening for aspirating a surgery site, which aspiration opening is connected to an aspiration channel extending through the housing and which aspiration channel is configured to be connected to a suction force source such as a pump or underpressure system in order to generate a suction force in the aspiration opening, **characterized in that** the handpiece comprises an operating element, wherein in a first operative mode of the operating element there is suction force in the aspiration opening and in a second operative mode of the operating element there is no suction force in the aspiration opening.

2. Handpiece according to claim 1, wherein the second operative mode is configured for the content of at least a part of the aspiration channel to flow back through the aspiration opening.

3. Handpiece according to claim 1 or 2, wherein the aspiration channel is formed by a flexible tube.

4. Handpiece according to any one of the foregoing claims, wherein the aspiration channel comprises a shut-off valve connected operatively to the operating element, which shut-off valve opens the aspiration channel in the first operative mode and closes the aspiration channel in the second operative mode.

5. Handpiece according to claims 3 and 4, wherein the shut-off valve presses the flexible tube shut from the outside in the second operative mode.

6. Handpiece according to any one of the foregoing claims, wherein in a third operative mode of the operating element there is no suction force in the aspiration opening and an internal volume of the aspiration channel which is connected to the aspiration opening in the third operative mode can be reduced for the purpose of the content of at least a part of the internal volume flowing back through the aspiration opening.

7. Handpiece according to claim 6, wherein the operating element comprises a part movable toward the aspiration opening for the purpose of reducing the internal volume of the aspiration channel, which is connected to the aspiration opening in the third operative mode.

8. Handpiece according to claim 7 in combination with claim 4 or 5, wherein the movable part is formed by an elongate operating part, which is arranged bendably on the operating element close to the shut-off valve and extends toward the aspiration opening.

9. Handpiece according to claim 8, wherein the elongate operating part has a curved contact surface, whereby the flexible tube can be pressed shut gradually from the shut-off valve toward the aspiration opening by bending of the elongate operating part.

10. Handpiece according to any one of the foregoing claims, wherein the handpiece also comprises at the distal outer end an irrigation opening for irrigating an operating site, which irrigation opening is connected to an irrigation channel extending through the housing and which irrigation channel is configured to be connected to an irrigation source such as a pump system in order to generate an irrigation flow in the irrigation opening.
